(19) Europäisches Patentamt
European Patent Office
Office européen des brevets

(11) Publication number: 0 034 174
B1

(12) # EUROPEAN PATENT SPECIFICATION

(45) Date of publication of patent specification: 08.05.85

(51) Int. Cl.$^4$: C 08 F 18/00

(21) Application number: 80901753.6

(22) Date of filing: 19.08.80

(86) International application number:
PCT/US80/01058

(87) International publication number:
WO 81/00570 05.03.81 Gazette 81/06

(54) **HYDROGEL IMPLANT ARTICLE AND METHOD.**

(30) Priority: 20.08.79 US 67630

(43) Date of publication of application:
26.08.81 Bulletin 81/34

(45) Publication of the grant of the patent:
08.05.85 Bulletin 85/19

(84) Designated Contracting States:
CH DE FR GB LI

(56) References cited:
GB-A-1 495 043
US-A-3 503 942
US-A-3 983 083
US-A-4 228 269

(73) Proprietor: RETINA FOUNDATION
20 Staniford Street
Boston, MA 02114 (US)

(72) Inventor: REFOJO, Miquel F.
16 Adams Street
Lexington, MA 02173 (US)

(74) Representative: Harvey, David Gareth et al
Graham Watt & Co. Riverhead
Sevenoaks Kent TN13 2BN (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 034 174 B1

## Description

Background of the invention

This invention relates to a new scleral buckle material and more generally to a new synthetic hydrophilic polymer for eye surgery and other *in vivo* implants.

Synthetic materials have several applications in ophthalmic medicine, including surgical implantation to create a scleral buckle to correct a condition known as retinal detachment, and as contact lenses. Silicone materials, both rubber and sponge, are known for use in scleral buckle procedures. Synthetic organic polymers with hydrophilic properties also have been used as scleral buckle materials, and certain species are the popular materials for contact lenses. Recent developments in correcting retinal detachment with scleral buckling are described in "Sutureless Scleral Buckling", G. A. Calabria, R. C. Pruett, M. F. Refojo, and C. L. Schepens, *Archives of Ophthalmology,* May 1970, Vol. 83, pp. 613—618; "Further Experience With Sutureless Scleral Buckling Materials", G. A. Calabria, R. C. Pruett, and M. F. Refojo, *Archives of Ophthalmology,* July 1971, Vol. 86, pp. 77—81; and "Experimental Scleral Buckling With A Soft Xerogel Implant", M. F. Refojo and H. S. Liu, *Ophthalmic Surgery,* December 1978, Vol. 9, No. 6, pp. 43—50. Materials for contact lenses, and particularly for soft contact lenses, are described in "Contact Lens Materials", M. F. Refojo, *International Ophthalmology Clinics,* Spring 1973, Vol. 13, No. 1, pp. 263—277; Contact Lenses", M. Refojo, *Encyclopedia of Polymer Science And Technology,* Supplement Volume 1, pp. 195—219; and "Contact Lenses", M. F. Refojo, *Encyclopedia of Chemical Technology,* Volume 6, Third Edition, pp. 720—742. The polymer chemistry of certain synthetic hydrogels is described in *Soft Contact Lenses: Clinical And Applied Technology,* M. Ruben, Editor, Published by John Wiley & Sons, Chapter 3, pp. 19—38.

Materials for these ophthalmologic applications are to be non-toxic and otherwise tolerated without causing tissue inflamation or other rejection mechanisms, and they are to be relatively nonabsorbable. The materials are also to be capable of sterilization without deterioration, and are to be permeable to oxygen, water and low molecular-weight water-soluble substances. Implant materials also are often to be non-biodegradable. Another desired property is that the implant material be capable of being cast or otherwise formed into an article of specific configuration, which the article retains. In addition, material for a scleral buckle preferably is soft, pliable and elastic; a specific objective is that it be capable of being compressed by overlying sutures without cutting through the implant or of being applied with sutureless techniques. Materials for ophthalmic surgery in addition are often desired to absorb antibiotics and other drugs for prolonged release after surgical implantation. In addition, it is desired that surgically implantable materials have pores of such small size that they do not form sites for infection by bacterial or other pathogens.

Known materials meet numerous of these properties, but all too often the fulfillment of some properties is attendant with deficiencies with regard to other properties. For example, methacrylate hydrogels known for use as scleral buckles have the disadvantage of being hard and stiff when dry; they become soft and pliable only when wet. The known silicone rubber sponges used in scleral buckle surgery is considered to have pores sufficiently large to provide a site for bacterial infection.

It is accordingly an object of this invention to provide an improved hydrophilic gel, or hydrogel, for surgical implantation. An experimental hydrogel implant article for scleral buckling has been disclosed in a paper in Ophthalmic Surgery, Vol. 9, No. 6, December 1978, pp. 43—50, made from a hydroxyalkyl acrylate homopolymer. This homopolymeric article is soft both when dehydrated and when hydrated, but its mechanical properties are not as satisfactory as desired, and the paper reports special precautions must be taken to prevent its fragmentation when sutured.

A more particular object is to provide an implant hydrogel which attains the properties discussed above to a greater extent than previously available, and further to provide such a hydrogel suitable for ophthalmic use.

Another object of the invention is to provide an implant hydrogel having improved properties in terms of softness, in terms of elasticity, and in terms of resistance to tensile rupture. More specific objects are that the hydrogel be soft, pliable and elastic when dry as well as when wet, and furthermore that it be sufficiently tough, for example, to hold a surgical suture. Thus the objects of the invention include the provision of a scleral buckle hydrogel which is soft and elastic when wet as well as when dry, and which has sufficient resistance to tensile rupture to hold a suture.

These objects are met by a shape-retaining hydrogel surgical implant article according to the invention which is a hydroxyalkyl acrylate polymer and is soft and pliable both when wet and when dry, the article being characterised in that it comprises a copolymer solution polymerized in an organic solvent from a mixture of at least 50% by volume of an alkyl acrylate and at least 30% by volume of 2-hydroxyethyl acrylate, with a cross-linking monomer being present in an amount of up to 4% by volume of the 2-hydroxyethyl acrylate, the alkyl acrylate having for its alkyl group one containing 1 to 5 carbon atoms.

It has been found that acrylate hydrophilic gels can be prepared with properties superior to those previously assumed. In particular, it was considered in the prior art that methacrylate hydrogels were preferred over those of acrylate derivatives. It is understood that this preference stemmed from the view that the methacrylate

hydrogels were more resistant than acrylates to hydrolysis and to biodegradation, and were considered to have better stability and particularly resistance to thermodegradation, especially such as encountered in sterilizing with heat as in an autoclave. Contrary to this view, this invention provides acrylate hydrogels that function well as implants without exhibiting these supposed shortcomings and, further, that have advantages over prior materials.

The hydrogel surgical implant article which the invention provides comprises a random copolymer, with slight cross-linking, of 2-hydroxyethyl acrylate with an alkyl acrylate monomer of which the alkyl group is one having one to five carbon atoms. Hence, nonlimiting examples of common alkyl acrylate monomers are methyl acrylate and n-butyl acrylate. The monomers are polymerized by the solution polymerization technique, with simultaneous cross-linking, in an organic solvent, an example of which is ethylene glycol. In addition to the monomers, a cross-linking agent and a free radical initiator are present in the prepolymer solution. Typical cross-linking agents are ethylene glycol diacrylate, ethylene glycol dimethacrylate, di- or triethylene glycol diacrylates and divinyl benzene. Sufficient cross-linking agent may be present as an impurity in the monomer, especially in the 2-hydroxyethyl acrylate. The quantity of cross-linking agent present in the monomer can be determined in advance with standard techniques such as chromatography, and an appropriate additional quantity introduced prior to polymerization as needed. Examples of the free radical initiator are isopropyl percarbonate, benzoyl peroxide, and azobisisobutyronitrile. Practice of the invention is not limited to such an initiator, and can for example instead use a redox catalyst, such as mixtures of ammonium persulfate and sodium metabisulfate.

The hydrogel implant polymerized from these monomers has the following formula in each cross-linked random copolymer

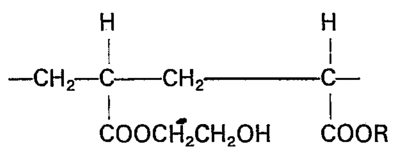

where R is an alkyl group selected from the methyl, ethyl, propyl, butyl, and pentyl groups.

A preferred method of preparing the hydrogel surgical implant article which the invention provides includes the steps of placing a solution of the monomers with the selected initiator and selected cross-linking agent in a mold which is then sealed. The mold typically is siliconized glass tubing or of a plastic such as polyethylene or polypropylene. The prepolymer solution is frozen in dry ice in the mold, to remove gas, prior to sealing the mold closed. The monomers are polymerized in the mold at a controlled temperature of about 30°C to 50° Centigrade for twelve to twenty-four hours. This is followed by curing in an oven at about 60° to 80° Centigrade for five to ten hours with a further, shorter, e.g. one hour, curing step at about 90° Centigrade. In one example, the polymerization is carried out by placing the mold in a water bath maintained at 37°C Centrigrade for twelve to fourteen hours, and the curing employs a first step at 70° Centigrade for seven hours and a second step at 90° for one hour.

Upon completion of the polymerization, including curing, the resultant hydrophilic gel copolymer is removed from the mold and treated to exchange the polymerization solvent and residual monomers as well as any other unpolymerized material, with an aqueous solution such as water or isotonic saline. This washing and exchange typically includes a continuous extraction process and is continued until no measurable amount of leachable impurities remains in the copolymer. The resultant clean copolymer is sterilized by autoclaving, after which it typically is stored in isotonic saline or other physiological solution until ready for use.

The hydrogel obtained in the foregoing manner typically has a Shore Durometer (Type A-2) hardness in a wet state in the range of five to thirty with a typical and preferred value in the order of ten. The desired degree of hardness is obtained by selecting the amount of cross-linking agent present in the prepolymer solution. Typically, the cross-linking agent is present in an amount from 0.2% to as much as 4% of the volume of hydroxyethyl acrylate monomer.

The implant hydrogel thus is caracterized by being soft, pliable and elastic both when wet with aqueous solution as well as when dry. These properties distinguish it from many hydrogel formulations known in the art which are soft, elastic and pliable when wet, but are significantly less so when dry. This advantage of the acrylate copolymer which the invention provides facilitates its use as an implant. For example, it facilitates implanting the hydrogel as a scleral implant in retinal detachment surgery.

The copolymer is also characterized by a pore size in the molecular range with maximum pore diameter in the order of twenty to thirty Angstrom units (2 to 3 mm). Such sub-microscopic pore are desirable because they are too small to receive bacteria and like pathogens, while yet being sufficient to enable the hydrogel to absorb hydrophilic antibiotics or other aqueous drugs. An antibiotic typically is introduced by placing the hydrogel in an aqueous solution of the antibiotic shortly, e.g. one-half hour, before surgically implanting the hydrogel. The implanted hydrogel gradually releases the absorbed antibiotic over a relatively prolonged period after surgery, thereby minimizing the post-operative infection. The acrylic hydrogel typically absorbs about 15% to 60% by weight of water or other aqueous solution, which as noted can include drugs. Other features of the implant hydrogel are high tissue tolerance, stability under heat sterilization,

stability against biodegradation after surgical implantation, and relatively high tensile strength.

The nature of the invention and the objects it attains will now be described further with reference to detailed examples of the foregoing practice.

Example I

An implant of methyl acrylate copolymer for ophthalmic surgical implantation was prepared as follows. Commercial grades of methyl acrylate (MA) and of 2-hydroxyethyl acrylate (HEA) were redistilled and analyzed by gas chromatography. The MA was determined to be pure, and the HEA contained (by volume) 0.6% acrylic acid, 1.0% ethylene · glycol, and 1.2% ethylene glycol diacrylate. The redistilled monomers were mixed in the following proportions in six milliliters ethylene glycol as a solvent: HEA, 3 milliliters, and MA, 7 milliliters; plus 12 to 14 milligrams of isopropyl percarbonate as a polymerization initiator, i.e. free radical initiator. The 1.2% ethylene glycol diacrylate present in the distilled HEA monomer was sufficient to function as a crosslinking agent. To polymerize the mixture, the solution was placed in a mold of polypropylene tubing having a wall thickness of one millimeter and an inner diameter of six millimeters, and the tubing ends sealed after the liquid was frozen to remove gas bubbles. The sealed tube was placed in a water bath, and the polymerization reaction carried out for about twelve hours at 37° Centigrade, followed by a first curing step of seven hours in an oven at 70° Centigrade, and a second curing step at 85° Centigrade for one further hour. The copolymer was removed from the mold and washed thoroughly in distilled water at room temperature for several weeks, with repeated changes of water. The copolymer was next extracted in a soxhlet with methanol until negligible amounts of extractables were detected spectrophotometrically. The methanol which the polymer absorbed was then exchanged with distilled water by placing the polymer in water which was repeatedly changed. The hydrated polymer has more uniform dimensions and hardness when it is dried after replacement of the methanol, and then rehydrated. The resultant hydrated polymer was placed for sterilization and storage in a glass vial with isotonic sodium chloride solution, and the sealed vial sterilized in a steam autoclave for twenty minutes at 120° Centigrade.

The physical properties and occular tolerance of the methyl acrylate copolymer implant prepared in this manner are as follows. The copolymer is insoluble, but swells in water and other solvents. At equilibrium swelling in water or in physiological saline solution, it absorbs about 15% by wet weight of the liquid, and is white, opaque, and soft having a hardness of 10 (Shore Durometer Hardness Type A-2). When dry, the copolymer implant is soft, pliable, elastic and optically transparent with a Durometer hardness of 54 (Shore Durometer Type A-2). The copolymer implant also absorbs hydrophilic antibiotics that are released upon implantation.

Ocular tolerance and irritation for the copolymer implant were studied in rabbits by ocular irritation tests with extractibles from the implant, and by implant tolerance tests in the anterior chamber and at the episclera. The ocular irritation tests revealed no ocular irritation or congestion during or after a test period of four days. There was no difference in appearance between the test eyes and the control eyes. With regard to ocular tolerance to implants, rabbit eyes with anterior chamber implants initialled revealed a localized fibrinous transudate covering the implant material but which cleared completely within forty-eight hours, after which the implant was seen over a normal iris with clear cornea. In eyes with episcleral implants, the conjunctiva initially revealed localized congestion but with no subsequent signs of irritation, and the cornea was clear and the conjunctiva congestion subsided within three days of the implant operation. Macroscopic examination of enucleated eyes revealed no inflammatory changes. Histologically, eyes with anterior chamber implants revealed normal corneal endothelium and normal smooth iris surface. Similarly, in those with episcleral explants, the underlying sclera and overlying conjunctiva appeared normal except for mild condensation of fibrous tissue around the implant.

The copolymer article was also surgically implanted in rabbits as a scleral buckle by suturing it over the sclera. Post-operative examination and eyes enucleated from animals at three weeks and at three months following the implant surgery revealed no evidence of infection, rejection, or cutting through the sclera in any eye. No biodegradation of an implant was apparent even after one year.

From these and other tests it was determined that the new alkyl acrylate implant is well tolerated by ocular tissues, has good resistance to sutures, and has ideal softness for scleral indentation in retina detachment surgery.

Example II

In contrast to polymerizing methyl acrylate in essentially a two-to-one volume proportion to hydroxyethyl acrylate as in Example I, a hydrogel implant according to the invention was prepared using essentially equal volumes. For this example, the redistilled methyl acrylate and 2-hydroxyethyl acrylate monomers described in Example I, but each in 5 milliliter quantities, were mixed in solution with 5 milliliters of ethylene glycol to which 10.8 milligrams isopropyl percarbonate were added. Again, sufficient ethylene glycol diacrylate was present as an impurity. The solution was polymerized, cured and washed as in Example I. The resultant dried copolymer is transparent, pliable, strong and elastic, but with slow elastic recovery. The Durometer hardness when dry is 55 (Shore Durometer A-2). Upon rehydration, the polymer absorbs 30% water by

volume and is transparent, very soft (Durometer hardness of 0.5 when hydrated without prior drying, and a somewhat higher value when dried and then rehydrated), and elastic.

Other cross-linked copolymers of 2-hydroxyethyl acrylate and methyl acrylate can be obtained with diverse compositions of the prepolymer mixture. A wide range of hydration and softness of the rehydrated polymer is obtained. The common feature of these copolymers is that they are soft and pliable in the dry state, and softer and more elastic in the hydrated state.

Example III

A butyl acrylate hydrophlic gel implant is prepared according to the invention with 5 milliliters HEA monomer, 6 milliliters n-butyl acrylate (BA) monomer, 5 milliliters of ethylene glycol solvent, and 11.2 milligrams of isopropyl percarbonate initiator. The monomers are redistilled, and the solution polymerized, as in Example I, with curing and washing to remove residuals as also described with reference to Example I. The cross-linking agent is ethylene glycol diacrylate present at about 2% as an impurity in the HEA monomer. The resultant copolymer when dry is soft, transparent, elastic and sticky, with a Durometer hardness of 12. Upon hydration, the polymer is transparent with good tensile strength, absorbs 11.2% water by wet weight, and has a Durometer measure of 2.6.

Other copolymers of HEA and BA can be made by varying the proportions of these ingredients in the prepolymer mixture to obtain a variety of random cross-linked copolymers which are soft and pliable in the dry state, and softer and more elastic in the hydrated state.

Example IV

Some crosslinked copolymers of HEA and of alkyl acrylates in accordance with this invention, when hydrated in water or in a physiological saline solution, are opaque or at least translucent. This is the case with the polymers obtained according to Examples I and III. For most implant applications, such as for scleral buckling in retinal detachment surgery, the optical properties of the implant are irrelevant. However, for intraocular implants, transparency is of course essential.

Some crosslinked copolymers of this invention have been found to have excellent optical properties as well as high resistance to rupture. Copolymers of this kind are given in Example II and in the following further example.

The polymerization and simultaneous crosslinking of the monomers given in Example I were carried out under the same conditions as given in that example, but in the proportions of HEA (with 1.2% ethylene diacrylate) 4 ml, MA 6 ml in solution in 5 ml of ethylene glycol, with 10.76 mg of isopropyl percarbonate as polymerization initiator. The resulting polymer was water washed, methanol extracted, rehydrated, and dried, Upon rehydration in distilled water, it yielded a transparent, elastic, strong, hydrophilic material of wet durometer 12.9 and hydration 19.8%. Hydrated in physiological saline solution (0.9% sodium chloride), the durometer reading was 13 and the hydration 17.9%.

The foregoing description and examples show that one can obtain hydrated alkyl acrylate copolymer implants with diverse degrees of softness and of elasticity by increasing the proportion of ethylene glycol diacrylate or adding other crosslinking agents to the prepolymer mixture. Nonlimiting examples of such agents include ethylene glycol dimethylacrylate, diethylene glycol diacrylates, triethylene glycol diacrylates, and divinyl benzene. The prepolymer mixture can have countless combinations and proportions of monomers, with different dilutions in ethylene glycol and other suitable solvents, nonlimiting examples of which include diethylene glycol and ethanol. The apparent materials of choice, however, include those described in the foregoing specific examples.

The hydrogel surgical implant articles prepared according to the foregoing examples are molded, or cast, in the desired configuration. The copolymer in addition can be cut to a selected size and shape. In each instance, the implant article elastically retains its shape, even after heat sterilization, as by autoclave.

The alkyl acrylate copolymer implant of the invention is considered to have several advantages over prior art materials that are in use and particularly over silicone sponge as currently used in the treatment of retinal detachment. These advantages include the finding that the alkyl acrylate implant, unlike silicone sponge, does not have macroscopic pores but rather has microscopic pores and hence reduces chances of infection. Other advantages are that the implant has molecular size porosity and is hydrophilic so that it can absorb aqueous antibiotics and other aqueous drugs and act as a depot for the sustained delivery of such absorbed drugs for protecting the surgical field against early postoperative infection. In addition, the new implant, like the prior art silicone sponge but unlike prior gelatin implants, is nonabsorbable. A further advantage is that the alkyl acrylate implant is soft when dry and softer when hydrated, so it can be implanted in the fully hydrated state to obtain the required buckle during surgery or implanted in the dehydrated state to obtain a higher buckle upon *in situ* hydration. In contrast to silicone rubber, the new implant material is not electrostatically charged and therefore does not attract lint and dust, which can obviously cause postoperative complications. A further advantage is that the alkyl acrylate implant is tolerated by ocular tissue at least as well as solid silicone rubber.

It will thus be seen that the implant of the invention provides a combination of a significant number of desired properties, and that the objects set forth above, among those made apparent from the preceding description, are efficiently attained. Since certain changes may be made in

the above composition of matter, in the carrying out of the foregoing method of its preparation, and in the resultant implant article as set forth, without departing from the scope of the invention, it is intended that all matter contained in the above description be interpreted as illustrative and not in a limiting sense.

It is also to be understood that the following claims are intended to cover all of the generic and specific features of the invention herein described, and all statements of the scope of the invention which, as a matter of language, might be said to fall therebetween.

**Claims**

1. A shape-retaining hydrogel surgical implant article, which is a hydroxyalkyl acrylate polymer and is soft and pliable both when wet and when dry, characterised in that it comprises a copolymer solution polymerized in an organic solvent from a mixture of at least 50% by volume of an alkyl acrylate and at least 30% by volume of 2-hydroxyethyl acrylate, with a cross-linking monomer being present in an amount of up to 4% by volume of the 2-hydroxyethyl acrylate, the alkyl acrylate having for its alkyl group one containing 1 to 5 carbon atoms.

2. An implant article according to claim 1, characterised in that the copolymer-producing mixture contains approximately equal amounts by volume of alkyl acrylate and 2-hydroxyethyl acrylate.

3. An implant article according to claim 1, characterised in that the copolymer-producing mixture contains twice as much alkyl acrylate by volume than 2-hydroxyethyl acrylate.

4. An implant article according to claim 1, 2 or 3, characterised in that the monomers are polymerized in a solution of ethylene glycol.

5. An implant article according to any preceding claim, further characterised by a Shore Durometer Type A-2 hardness of up to 55 when dry.

6. An implant article according to any of claims 1 to 5, characterised in that said alkyl acrylate is selected from methyl acrylate and n-butyl acrylate.

7. An implant article according to any preceding claim, further characterised as a hydrogel of said copolymer and an aqueous liquid.

8. An implant article according to claim 7, characterised in that said aqueous liquid includes an antibiotic.

9. A method of preparing a shape-retaining hydrogel implant article according to claim 1, the method comprising the successive steps of:

A. mixing the 2-hydroxyethyl acrylate and the alkyl acrylate in a water-soluble organic solvent with the cross-linking agent and with the free radical initiator,

B. placing said solution in a sealed mold,

C. subjecting the monomers to solution polymerization and crosslinking in said mold under controlled temperature,

D. curing the copolymer in the mold,

E. removing the resultant copolymer from the mold, and

F. extracting residues and solvent from said copolymer.

**Revendications**

1. Article d'implant hydrogel chirurgical conservant sa forme, qui est un polymère d'acrylate d'hydroxyalkyle et qui est mou et pliable, à la fois lorsqu'il est mouillé et lorsqu'il est sec, caractérisé en ce qu'il comprend un copolymère polymérisé en solution dans un solvant organique à partir d'un mélange d'au moins 50% en volume d'un acrylated alkyle et au moins 30% en volume d'acrylate de 2-hydroxyéthyle, avec un monomère de réticulation présent en une quantité jusqu'à 4% en volume de l'acrylate de 2-hydroxyéthyle, l'acrylate d'alkyle ayant pour son groupe alkyle un groupe contenant de 1 à 5 atomes de carbone.

2. Un article d'implant selon la revendication 1, caractérisé en ce que le mélange produisant le co-polymère contient environ des quantités égales en volume d'acrylate d'alkyle et d'acrylate de 2-hydroxyéthyle.

3. Un article d'implant selon la revendication 1, caractérisé en ce que le mélange produisant le copolymère contient deux fois plus d'acrylate d'alkyle en volume que d'acrylate de 2-hydroxyéthyle.

4. Un article d'implant selon la revendication 1, 2 ou 3, caractérisé en ce que les monomères sont polymérisés dans une solution d'éthylène-glycol.

5. Un article d'implant selon l'une quelconque des revendications précédentes caractérisé en outre par une dureté sur Duromètre Shore de type A-2 jusqu'à 55 lorsqu'il est sec.

6. Un article d'implant selon l'une quelconque des revendications 1 à 5, caractérisé en ce que cet acrylate d'alkyle est choisi entre l'acrylate de méthyle et l'acrylate de n-butyle.

7. Un article d'implant selon l'une quelconque des revendications précédentes caractérisé en outre comme un hydrogel de ce co-polymère et d'un liquide aqueux.

8. Un article d'implant selon la revendication 7, caractérisé en ce que ce liquide aqueux comprend un antibiotique.

9. Une méthode pour préparer un article d'implant hydrogel conservant sa forme selon la revendication 1 la méthode comprenant les étapes successives de:

A. mélanger l'acrylate de 2-hydroxyéthyle et l'acrylate d'alkyle dans un solvant organique soluble dans l'eau avec l'agent de réticulation et avec l'initiateur de radicaux libres,

B. disposer cette solution dans un moule scellé,

C. soumettre les monomères à une polymérisation en solution et réticulation dans ce moule sous température contrôlée,

D. durcir le copolymère dans le moule,

E. enlever le copolymère résultant du moule, et

F. extraire les résidus et solvant de ce copolymère.

**Patentansprüche**

1. Formhaltiger chirurgischer Hydrogel-Implantat-Artikel aus einem Hydroxyalkylakrylatpolmeren, der sowohl im feuchten als auch im trockenen Zustand weich und beigsam ist, dadurch gekennzeichnet, daß er von einem Copolymeren gebildet ist, das in einem organischen Lösungsmittel aus einem Gemisch von zumindest 50 Vol.-% eines Alkylakrylats und zumindest 30 Vol-% 2-Hydroxyäthylakrylat lösungspolymerisiert ist, wobei ein Vernetzungsmonomeres in einer Menge von bis zu 4 Vol.-% des 2-Hydroxyäthylakrylats vorhanden ist und das Alkylakrylat als Alkylgruppe eine Gruppe mit 1 bis 5 Kohlenstoff-Atomen hat.

2. Implantat-Artikel nach Anspruch 1, dadurch gekennzeichnet, daß das copolymerbildende Gemisch annähernd gleiche Volumenanteile des Alkylakrylats und des 2-Hydroxyäthylakrylats enthält.

3. Implantat-Artikel nach Anspruch 1, dadurch gekennzeichnet, daß das copolymerbildende Gemisch volumenbezogen zweimal soviel Alkylakrylat wie 2-Hydroxyàthylakrylat enthält.

4. Implantat-Artikel nach Anspruch 1, 2 oder 3, dadurch gekennzeichnet, daß die Monomeren in einer Äthylenglykollösung polymerisiert sind.

5. Implantat-Artikel nach einem der vorhergehenden Ansprüche, gekennzeichnet durch eine Shore-Härte von bis zu 55 im trockenen Zustand (Shore-Härtemesser Typ A-2).

6. Implantat-Artikel nach einem der Ansprüche 1 bis 5, dadurch gekennzeichnet, daß das Alkylakrylat aus Methylacrylat und Butylakrylat ausgewählt ist.

7. Implantat-Artikel nach einem der vorhergehenden Ansprüche, dadurch gekennzeichnet, daß es ein Hydrogen des genannten Cpolymeren und einer wäßrigen Flüssigkeit ist.

8. Implantat-Artikel nach Anspruch 7, dadurch gekennzeichnet, daß die wäßrige Flüssigkeit ein Antibiotikum enthält.

9. Verfahren zum Herstellen eines formhaltigen Hydrogel-Implantat-Artikels nach Anspruch 1, gekennzeichnet durch die nachstehenden aufeinanderfolgenden Verfahrensschritte:

A. das 2-Hydroxyäthylakrylat und das Alkylakrylat werden in einem wasserlöslichen organischen Lösungsmittel mit dem Vernetzungsmittel und mit dem freien Radikalinitiator gemischt,

B. die Lösung wird in eine geschlossene Form gebracht,

C. die Monomeren werden der Lösungspolymerisation und Vernetzung in der Form bei kontrollierter Temperatur ausgesetzt,

D. das Copolymere wird in der Form gehärtet,

E. das copolymere Erzeugnis wird aus der Form genommen und

F. Rückstände und Lösungsmittel werden aus dem Copolymeren extrahiert.